(19) Europäisches Patentamt
European Patent Office
Office européen des brevets

(11) Publication number: **0 102 476**
**B1**

(12) **EUROPEAN PATENT SPECIFICATION**

(45) Date of publication of patent specification: **05.11.86**

(21) Application number: **83106543.8**

(22) Date of filing: **05.07.83**

(51) Int. Cl.$^4$: **A 61 K 31/16,** A 61 K 31/135, A 61 K 31/41, C 07 C 147/12, C 07 D 257/04 // C07C147/14, C07C149/42

(54) **Method of modulating the immune response system in mammals.**

(30) Priority: **06.08.82 US 405666**
**25.08.82 US 411399**

(43) Date of publication of application:
**14.03.84 Bulletin 84/11**

(45) Publication of the grant of the patent:
**05.11.86 Bulletin 86/45**

(84) Designated Contracting States:
**AT BE CH DE FR GB IT LI NL SE**

(56) References cited:
**FR-A- 868 337**
**GB-A- 841 701**
**US-A-2 366 664**
**US-A-3 753 679**

**CHEMICAL ABSTRACTS, vol. 41, no. 7, 10th April 1947, column 2164e, Columbus, Ohio, USA B.L. FREEDLANDER et al.: "Derivatives of diphenyl sulfone, related sulfoxides, and sulfides in experimental tuberculosis"**

(73) Proprietor: **AMERICAN CYANAMID COMPANY**
**1937 West Main Street P.O. Box 60**
**Stamford Connecticut 06904 (US)**

(72) Inventor: **Lang, Stanley Albert, Jr.**
**7 Colony Drive**
**Blauvelt New York 10913 (US)**
Inventor: **Fields, Thomas Lynn**
**62 Amory Avenue**
**Pearl River New York 10965 (US)**
Inventor: **Wilkinson, Raymond George**
**7 Surrey Lane**
**Montvale New Jersey 07645 (US)**
Inventor: **Kang, Soon Mok**
**400 Knickerbocker Road**
**Dumont New Jersey 07628 (US)**
Inventor: **Lin, Yank-I**
**4 Pelham Court**
**Nanuet New York 10954 (US)**

(74) Representative: **Wächtershäuser, Günter, Dr.**
**Tal 29**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 102 476 B1

Courier Press, Leamington Spa, England.

**Description**

This invention is concerned with a novel method of modulating the immune response system in a warm-blooded animal which comprises administering to said animal an effective amount of a compound of the formula:

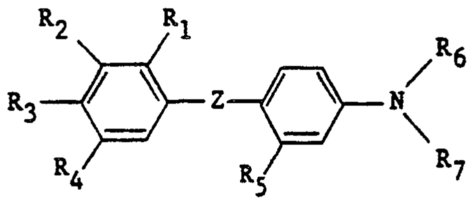

wherein $R_1$ is hydrogen, nitro or chloro; $R_2$ is hydrogen or chloro; $R_3$ is hydrogen, fluoro, chloro, bromo, nitro, alkoxy having up to 3 carbon atoms, acetylamino, B-hydro-xyethylamino or $-NHCOCH_2NHCH_3$; $R_4$ is hydrogen or chloro; $R_5$ is hydrogen or chloro; $R_6$ is hydrogen or alkyl having up to 3 carbon atoms; $R_7$ is hydrogen, alkyl having up to 3 carbon atoms,

$-CH_2CN_2$, $-COCH_2NH_2$, $-COCH_2NHCH_3$, $-COCH_2Cl$, $-COCH_2CH_2Cl$,

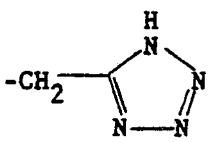 or 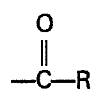

wherein R is alkyl having up to 4 carbon atoms such as methyl, *iso*propropyl, *n*-butyl, *tert*-butyl, etc.; and Z is thio (—S—), sulfinyl (—SO—) or sufonyl ($SO_2$—); with the proviso that the phenyl ring bearing the $R_1$, $R_2$, $R_3$ and $R_4$ substituents is mono-substituted except for polychloro substitution; together with the pharmacologically acceptable acid-addition salts thereof; in association with a pharmaceutically acceptable carrier.

In addition, this invention is concerned with novel compounds of the formula:

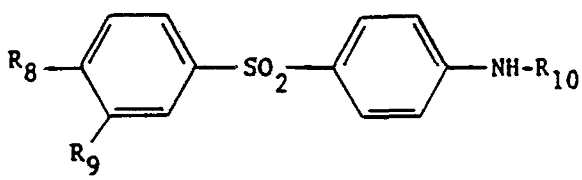

wherein $R_8$ is hydrogen, fluoro, chloro, or bromo and $R_9$ is hydrogen or chloro with the proviso that one of $R_8$ and $R_9$ must be hydrogen but $R_8$ and $R_9$ may not both be hydrogen; and $R_{10}$ is selected from the group consisting of

$-COCH_2NH_2$, 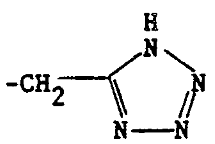

and $-CH_2CN$ together with their pharmaceutically acceptable salts.

The U.S. patent 2,366,664 discloses monoacyl-p,p'-diaminophenyl sulfones which are useful in the treatment of infectious diseases.

Chemical Abstracts Vol. 41, No. 7, 1947 reference No. 2164f discloses derivatives of diphenyl sulfones, related sulfoxides and sulfides for the experimental treatment of tuberculosis.

Certain of the active compounds of this invention may be prepared in accordance with the following reaction scheme:

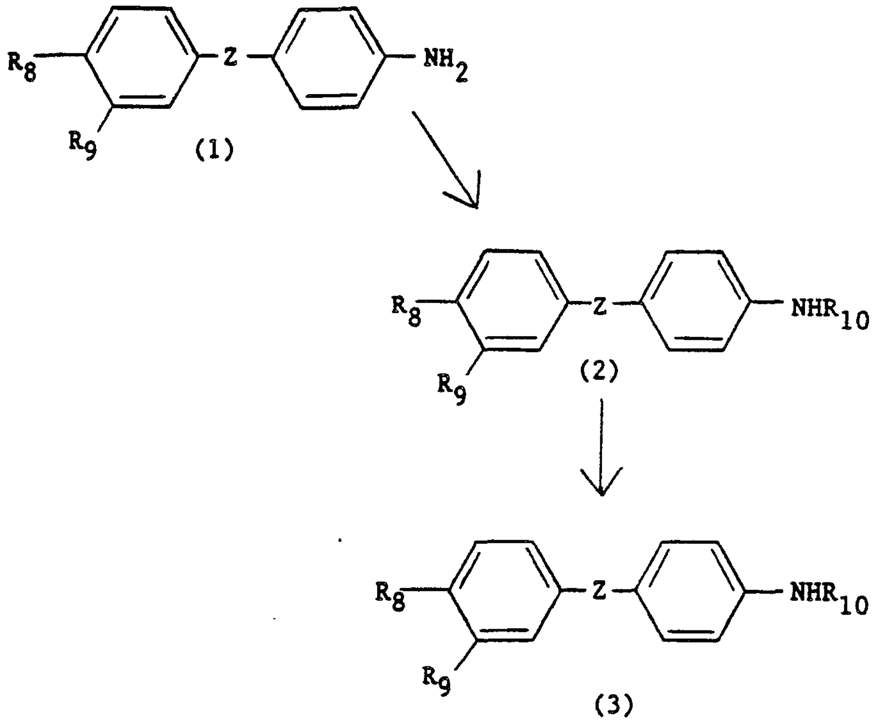

(1) (2) (3)

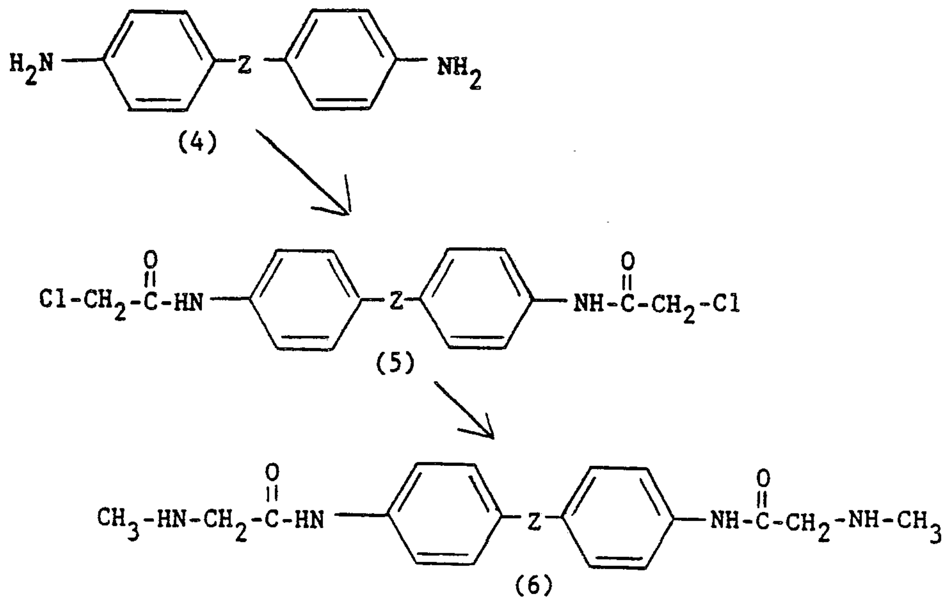

(4) (5) (6)

wherein $R_8$, $R_9$, $R_{10}$ and Z are as hereinabove defined. In accordance with the above reaction scheme, a 4-amino-substituted phenyl sulfide, sulfoxide or sulfone (1) is reacted with $R_{10}$-X where $R_{10}$ is $—COCH_2CH_2Cl$, $—COCH_2Cl$, $—CH_2CN$ or —COR and X is chloro or bromo in a solvent such as toluene at reflux for several hours. The solvent is evaporated and the product crystallized from a solvent such as ethanol giving (2). The compounds (2), where $R_{10}$ is $—COCH_2Cl$, may then be reacted with methylamine or ammonia to give the products (3) where $R_{10}$ is $—COCH_2NH_2$ or $—COCH_2NHCH_3$. The compounds (2) where $R_{10}$ is $—COCH_2Cl$ may also be treated with sodium azide to give azido derivatives which are then reduced with hydrogen sulfide and triethylamine or hydrogen and Raney Nickel to give the products (3) where $R_{10}$ is $—COCH_2Nh_2$. The compounds (2) where $R_{10}$ is $—CH_2Cn$ may be reacted with sodium azide to give the products (3) where $R_{10}$ is

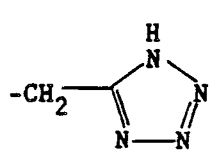

A *bis*(*p*-aminophenyl)sulfide, sulfoxide or sulfone (4) where Z is —S—, —SO— or —$SO_2$— is reacted with chloroacetyl chloride to give a *bis*[*p*-(chloroacetamido)phenyl]sulfide, sulfoxide or sulfone (5) which is then reacted with methylamine in ethanol at reflux for several hours giving (6) where Z is as described above.

Most of the active compounds of the present invention may be readily prepared in accordance with the following reaction scheme wherein $R_1$, $R_2$, $R_3$, $R_4$, $R_5$, $R_6$ and $R_7$ are as hereinbefore defined.

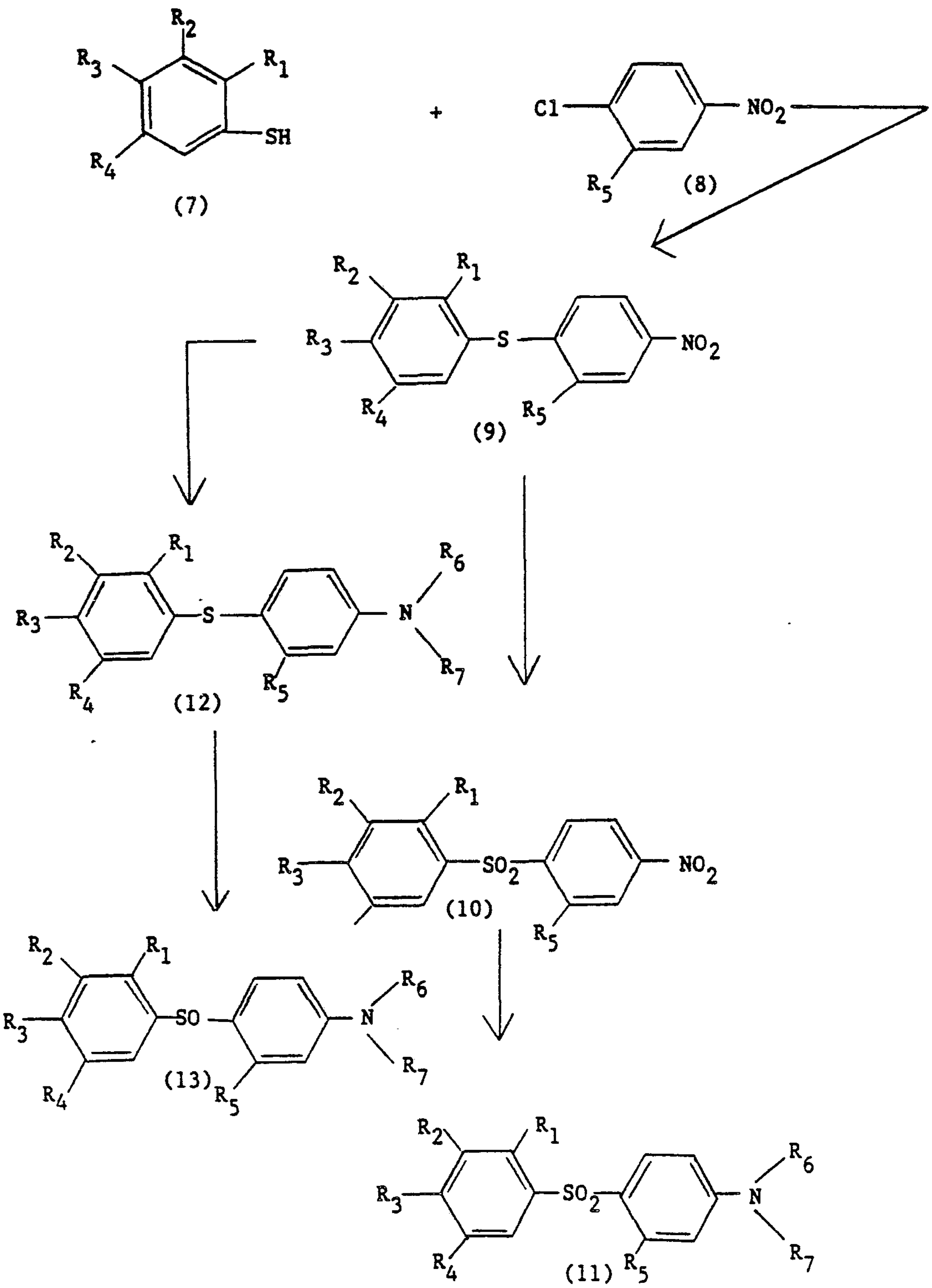

(7) (8) (9) (12) (10) (13) (11)

In accordance with the above reaction scheme, an appropriately substituted thiophenol (7) is condensed with a substituted schloro-nitrobenzene (8) in an approximately 50% aqueous-lower alkanol solvent at the reflux temperature (75°—100°C) thereof for about 4—20 hours in the presence of an acid acceptor such as pellet KOH in soda ash. Dilution of the reaction mixture with water precipitates the

corresponding substituted nitrodiphenyl sulfide product (9) which may be recrystallized from organic solvents such as ethanol, dimethylformamide, methylene chloride. Oxidation of the sulfide (9) to the corresponding sulfone (10) is accomplished by treatment with 30% aqueous hydrogen peroxide in glacial acetic acid as solvent at about 100°C for a few hours. Hydrogenation of the sulfone (10) is achieved in an inert solvent such as tetrahydrofuran or dioxane with Raney nickel catalyst at ambient temperatures for a few hours or until the stoichiometric amount of hydrogen is absorbed. Filtration of the reaction mixture followed by concentration of the filtrate provides the product (11) wherein $R_6$ and $R_7$ are both hydrogen. Hydrogenation of the sulfone (10) as just described but also in the presence of a carbonyl compound such as 37% formaldyhyde, acetaldyhyde, propionalydehyde or acetone provides the product (11) wherein $R_6$ and $R_7$ are lower alkyl. Hydrogenation of the sulfide (9) as described above hereinabove for the sulfone (10) provides the sulfide (12) wherein $R_6$ and $R_7$ are each hydrogen or lower alkyl. Oxidation of the sulfide (12) to the corresponding sulfoxide (13) is accomplished by treatment with 30% aqueous hydrogen peroxide in glacial acetic acid as solvent at about 50°C for a few hours.

The use of immunodulants and chemotherapeutic adjuvants constitutes a new therapeutic approach to the treatment of immune deficiencies and cancer and is based on the concept that there are distinctive antigens in or on most tumor cells (embryonal or transplantation antigens) that distinguish them from normal host cells. A majority of tumor immunologists favour the view that potentially malignant cells constantly arise but because of their "foreigness" they are normally eliminated by a competent humoral and cellular immune system. Occasionally however, tumor cells escape this immune surveillance and continue to reproduce cancer results. The reason for the failure of the normally efficient immune surveillance mechanisms are not fully understood but it is thought that the immune system becomes less effective with increasing age. It is depressed in certain genetic immuno-deficiency deseases, in various bacterial, fungal or vital infections, and in patients undergoing immuno-suppressive therapy. The growth of the neoplasm itself, as well as the various therapeutic modalities designed to treat the disease, e.g., cytotoxic chemotherapy and radiation, leads to a still greater depression of host resistance and results in an increased susceptability to both exogenous and endogenous infections and perhaps accounts for the re-initiation of tumor growth and metastasis which frequently follows treatment-induced tumor remission.

If depression of the immune system can result in the growth of malignancies, regulation of any facet of the immune response may help the host to eliminate residual cancer cells. Therefore, it is desirable to search for chemical agents (i.e. immunoregulants) capable of restoring and stimulating host immune defense mechanisms in order to overcome the deficiencies which account for susceptability to disease and failure to eradicate the cancer. Such immuno-regulating agents would likely be incapable of arresting the growth of a large tumor but their clinical utility would derive from their capacity to enhance normal immune surveillance mechanisms in patients whose tumor burden has been reduced by surgical, radiotherapeutic or chemotherapeutic methods.

Experimental studies in animal have demonstrated the antitumor potential of a number of immuno-regulants including live organisms of bacillus Calmett-Guerin (BCG), heat-killed cells of *Corynebacterium parvum.*, polynucleotides, and the anthelmintic drug, levamisole. These substances have been shown to stimulate cellular immunity and to produce tumor regression. Some successes have been claimed in early clinical trials with BCG against malignant melanoma and acute leukemia, and with levamisole against lung cancer and breast cancer. Although the antitumor effects produced by these agents have been promising, significant therapeutic benefits have yet to be realized. Since this is a new therapeutic approach, new drugs and methods of treatments must receive careful clinical evaluation in order to reveal their full potential.

Modern research is directed to the discovery of a drug similar to, but more potent than, known immuno-regulants such as levamisole that would be effective in the eradication of tumor cells when used in conjunction with standard therapeutic measures. Stimulators of host resistance may be detected in animal models that can, in fact, detect both immunostimulators and anticancer agents. Mice are put in a condition simulating immunodepression, common to cancer patients. This is accomplished by infecting mice with a leukemia virus which produces both leukemia and a disease-related immunodepression. Effective drugs are recognized by their ability to restore or enhance the antibody response in the experimental mice, or to inhibit tumor progression.

The active compounds and novel compositions of the present invention are active as immunomodulators when tested according to the following procedure, *Restoration of Antibody Formation in Mice with Rauscher Virus-Induced Leukemia.*

Infection of Balb/c mice with Rauscher leukemia virus (RLV) is characterized by: 1) a rapidly developing viremia, 2) suppression of the primary antibody response to antigens administered a few days after virus infection, 3) a progressive enlargement of the spleen (spenomegaly) and 4) death resulting from splenic rupture and hemorrhage. The protocol used to infect Balb/c mice with RLV and to test drugs for anticancer and/or immunostimulating activity is as follows:

Day 0:

Inject 0.2 ml of a 20% (w/v) RLV-infected spleen cell extract intraperitoneally (IP) into groups of 5 Balb/c mice. The spleen cell extract is prepared from mice infected with RLV 21 days previously.

Day +6, +7, +8:

Test compounds are administered orally or by IP injection, in 0.5 ml of normal saline containing 0.2% Noble agar.

Day =7:

Inject 0.5 ml. of IP of a thrice saline washed 10% suspension of sheep red blood cells (S—RBC).

Day 14:

Bleed mice from the retro-orbital sinus; pool blood from each group. Serum, harvested from pooled blood of each group of mice is stored at 4°C for 24 hours. Hemagglutinin tests are performed by standard procedures using a microtier technique. Acceptable hemagglutinin titer for leukemic (immunosuppressed) mice is = 1:128. The positive control compounds are Poly I:C (polyinosinic acid:polycytidylic acid) and Sarcosine administered intraperitoneally on days +6, +7, and +8. Acceptable positive control hemagglutinin titers are 4-fold higher than the titers obtained in leukemic control mice.

Typical compounds of this invention are active in this test, in that they produce a 4-fold or higher increase in hemagglutinin titer to sheep-RBC's, relative to the placebo treated, RLV-infected control mice. Results of this test appear in Table I below.

TABLE I

Antibody Restoration in Mice with Rauscher Virus-Induced Leukemia

| Compound | Dose (mg/kg) | Route | Fold increase in Serum Hemagglutinin Titer |
|---|---|---|---|
| 3-chloro-4′-(*p*-nitrophenylthio)-propionanilide | 1800 | Oral | 64 |
| 2-methylamino-4′-(*p*-nitrophenylthio)-acetanilide, hydrochloride | 200 | Oral | 8 |
| 2-chloro-4′-(*m*-chlorophenylsulfonyl)-acetanilide | 1800 | Oral | 8 |
| 2-amino-4′-(*m*-chlorophenylsulfonyl)-acetanilide | 400 | Oral | 16 |
| 4′-(*m*-chlorophenylsulfonyl)-2-methylamino-acetanilide | 800 | Oral | 4 |
| *bis*-[f-(2-methylaminoacetamido)-phenyl] sulfone | 1800 | Oral | 8 |
| 4′-(*p*-chlorophenylsulfonyl)-acetanilide | 100 | Oral | 16 |
| 2-amino-4′-(*p*-chlorophenylsulfonyl)-acetanilide hydrochloride | 100 | Oral | 4 |
| [p-(*p*-chlorophenylsulfonyl)anilino]-acetonitrile | 400 | Oral | 16 |
| 5-[[*p*-(*p*-chlorophenylsulfonyl)anilino]-methyl]-1H-tetrazole | 400 | Oral | 8 |
| 4-fluoro-4′-(2-aminoacetamido)-diphenylsulfone | 300 | Oral | 16 |
| N-[4-(4-fluorophenylsulfonyl)-phenyl]acetamide | 400 | Oral | 64 |
| N-[4-(4-bromophenylsulfonyl)-phenyl]acetamide | 200 | Oral | 16 |

TABLE I (Continued)

| Compound | Dose (mg /kg) | Route | Fold increase in Serum Hemagglutinin Titer |
|---|---|---|---|
| N-[4-(3-chlorophenylsulfonyl)-phenyl]acetamide | 100 | Oral | 4 |
| N-[4-[(4-fluorophenyl)sulfinyl]-phenyl]acetamide | 100 | IP | 4 |
| N-[4-[(4-methoxyphenyl)thio]-phenyl]acetamide | 400 | Oral | 4 |
| N-[4-[(4-methoxyphenyl)sulfinyl]-phenyl]acetamide | 400 | Oral | 4 |
| N-[4-[(4-chlorophenyl)sulfinyl]-phenyl]acetamide | 100 | IP | 4 |
| N-[4-[(4-chlorophenyl)thio]phenyl]-acetamide | 100 | IP | 16 |
| N-[4-[(4-bromophenyl)thio]phenyl]-acetamide | 100<br>200 | IP<br>Oral | 8<br>16 |
| N-4-[(4-bromophenylsulfinyl]phenyl]-acetamide | 400 | Oral | 8 |
| N-[4-[(4-fluorophenyl)sulfonyl]phenyl]-2-methylpropanamide | 200 | Oral | 16 |
| *p*-(*p*-fluorophenylsulfonyl)aniline | 100 | IP | 8 |
| *p*-(*o*-nitrophenylthio)aniline | 400 | Oral | 4 |
| *p*-(2,4,5-trichlorophenylsulfonyl)aniline | 400 | Oral | 4 |
| *p*-(*p*-bromophenylsulfonyl)aniline | 100 | IP | 8 |
| *p*-(2,4,5-trichlorophenylthio)aniline | 400 | Oral | 4 |
| *p*-(2,5-dichlorophenylthio)aniline | 400 | Oral | 16 |

TABLE I (Continued)

| Compound | Dose (mg/kg) | Route | Fold increase in Serum Hemagglutinin Titer |
|---|---|---|---|
| *p*-(*m*-chlorophenylsulfonyl)aniline | 400 | Oral | 8 |
| *p*-(3,4-dichlorophenylthio)aniline | 25 | Oral | 8 |
| *p*-(*p*-methoxyphenylsulfonyl)aniline | 400 | Oral | 16 |
| *p*-(3,4-dichlorophenylsulfonyl)aniline | 100 | IP | 8 |
| 3′-chloro-4-(*p*-chlorophenylsulfonyl)-aniline | 400 | Oral | 4 |
| *p*-(*p*-chlorophenylsulfonyl)aniline | 50 | IP | 16 |
| 4-acetylamino-4′-aminodiphenylsulfone | 100 | IP | 4 |
| *p*-[(*p*-fluorophenyl)sulfinyl]-N,N-dimethylbenzeneamine | 100 | IP | 4 |
| Poly I:C | 10 | IP | 16 |

The compounds of the present invention are effective as immunomodulators (that is, they modulate the immune response) when administered orally in amounts ranging from 5 mg to 400 mg per kilogram of body weight per day. A preferred dosage regimen for optimum results would be from 5 mg to 50 mg per kilogram of body weight per day, and such dosage units are employed that a total of from 350 mg to 3.5 grams of the active compound for a subject of about 70 kg of body weight are administered in a 24 hour period. This dosage regimen may be adjusted to provide the optimum therapeutic response. For example, several divided doses may be administered daily, or the dose may be proportionally reduced as indicated by the exigencies of the therapeutic situation. A practical advantage of this invention is that the active compound may be administered in any convenient manner as the oral or buccal routes.

The compounds of the present invention may be orally administered, for example, with an inert diluent or with an assimilable edible carrier, or they may be enclosed in hard or soft shell gelatin capsules, or they may be compressed into tablets. For orally therapeutic administration, the active compounds may be incorporated with excipients and used in the form of tablets, troches, capsules, elixirs, suspensions, syrups, wafers, and the like. Such compositions and preparations should contain at least 0.5% of active compound. The percentage of the compositions and preparations may, of course, be varied and may conveniently be between about 2% to about 60% of the weight of the unit. The amount of active ingredient is such therapeutically useful compositions is such that a suitable dosage will be obtained. Preferred compositions or preparations according to the present invention are prepared so that an oral dosage unit form contains between about 50 and 250 mg of active compound. The tablets, troches, pills and capsules may also contain the following: a binder such as gum tragacanth, acacia, corn starch or gelatin; excipients such as dicalcium phosphate; a disintegrating agent such as corn starch, potatoe starch and alginic acid; a lubricant such as magnesium stearate; and a sweetening agent such as sucrose; lactose or saccharin may be added or a flavouring agent such as peppermint, oil of wintergreen, or cherry flavoring. When the dosage unit form is a capsule, it may contain, in addition to materials of the above type, a liquid carrier such as a fatty oil. Various other materials may be present as coatings or to otherwise modify the physical form of the dosage unit. For instance, tablets, pills, or capsules may be coated with shellac, sugar, or both. A syrup or elixir may contain the active compound, sucrose as a sweetening agent, methyl and propylparabens as preservatives, a dye and flavoring such as a cherry or orange flavor. Of course, any

material used in preparing any dosage unit form should be pharmaceutically pure and substantially nontoxic in the amounts employed.

The invention will be described in greater detail in conjunction with the following specific examples.

Example 1

2-Chloro-4'-(*p*-nitrophenylthio)acetanilide

A mixture of 10.0 g or 4-amino-4'-nitrophenyl-sulfide and 4.5 g of chloroacetyl chloride in 150 ml of toluene was refluxed for 48 hours. The solvent was removed by evaporation and the residue crystallized from ethanol, giving 12 g of the desired product, mp 217—219°C.

Example 2

3-Chloro-4'-(*p*-nitrophenylthio)propionanalide

To a mixture of 5.0 g of 4-amino-4'-nitrodiphenylsulfide in 120 ml of toluene and 20 ml of dichloromethane was added 2.6 g of 3-chloropropionyl chloride in 15 ml of toluene, dropwise. The mixture was heated and stirred at reflux for 2 hours then the solvent was evaporated and the residue recrystallized from ethanol, giving 6.2 g of the desired product, mp 145—147°C.

Example 3

2-Methylamino-4'-(*p*-nitrophenylthio) acetanilide hydrochloride

A mixture of 3.0 g of 2-chloro-4'-(*p*-nitrophenylthio) acetanilide and a 4.0% aqueous solution of methylamine in ethanol was refluxed for 4 hours. The solvent was evaporated, the residue dissolved in dichloromethane and crystallized by the addition of hexane, giving 2.25 g of the desired product, mp 86—89°C.

Example 4

2-Chloro-4'-(*m*-chlorophenylsulfonyl)acetanilide

To a stirred mixture of 28.8 g of 3-chlorothiophenol and 23.3 g of sodium carbonate in 250 ml of water was added 31.5 g of 4-chloronitrobenzene followed by 200 ml of ethanol. The mixture was stirred at reflux for 4 hours and then filtered. The solid was treated with dichloromethane and water and then recrystallized from toluene, giving 42.1 g of 3-chlorophenyl-4'-nitrophenylsulfide.

A mixture of 40.0 g of 3-chlorophenyl-4'-nitro-phenylsulfide, 0.7 ml of 30% hydrogen peroxide and 230 ml of glacial acetic acid was hydrogenated in a Parr apparatus for 1.5 hours. The reaction mixture was filtered and the solid washed with two 150 ml portions of dioxane. Evaporation of the filtrate and wash gave a residue which was crystallized from ethanol, giving 43.4 g of 3-chlorophenyl-4'-nitrophenylsulfone.

A 12.5 g portion of 3-chlorophenyl-4'-nitrophenylsulfone in dioxane was hydrogenated in a Parr apparatus with Raney nickel catalyst, giving 4.9 g of 3-chlorophenyl-4'-aminophenylsulfone.

To a solution of 2.57 g of 3-chlorophenyl-4'-aminophenylsulfone and 3.0 ml of 2-methoxyethylether in 0.5 ml of dioxane was added 0.5 ml of chloroacetyl-chloride. The mixture was heated at 90—110°C for one hour. The solvent was evaporated and the residue recrystallized from cold hexane, then ethanol, giving 1.3 g of the desired product, mp 130—132°C.

Example 5

2-Amino-4'-(*m*-chlorophenylsulfonyl)acetanilide

A mixture of 2.0 g of 2-chloro-4'-(*m*-chlorophenylsulfonyl) acetanilide, 0.41 g of sodium azide, 20 ml of water and 40 ml of ethanol was refluxed for 3.5 hours and then evaporated to dryness. The residue was crystallized from ethanol, giving 1.2 g of 2-azido-4'-(*m*-chlorophenylsulfonyl) acetanilide, mp 125—126°C.

A mixture of 12 g of 2-azido-4'-(*m*-chlorophenylsulfonyl) acetanilide and 8 ml of Raney nickel in 150 ml of *p*-dioxane and 50 ml of ethanol was hydrogenated in a Parr shaker with an initial pressure of 30 psi of hydrogen for 2 hours. The mixture was filtered through a celite pad and the volatiles removed from the filtrate. The residue was crystallized from *p*-dioxane, giving 6.2 g of the desired product, mp 195—196°C.

Example 6

4'-(m-Chlorophenylsulfonyl)-2-methylaminoacetanilide

A mixture of 6.0 g of 2-chlorophenyl-4'-(m-chloro-phenylsulfonyl)acetanilide, 1100 ml of methyamine and 20 ml of ethanol was refluxed for 3.5 hours. The solvent was evaporated and the residue partitioned between dichloromethane and water. The dichloromethane portion givng 2.48 g of the desired product, mp 110—112°C.

Example 7

Bis-[p-(2-methylaminoacetamido)phenyl]sulfone

To a stirred solution of 24.83 g of bis-(p-amino-phenylsulfone in 100 ml of dioxane and 200 ml of dimethoxyethyl ether at 50°C was added a 1:1 mixture of chloro-acetylchloride and dimethoxyethyl ether. The mixture was heated at 90°C for 3.5 hours and then allowed to stand at ambient temperature. The solid was collected and crystallized giving 10.2 of bis[(p-(chloroacetamino)-phenyl]sulfone.

A mixture of 5.0 g of bis[(p-(chloroacetamido)-phenyl] sulfone and 100 ml of methylamine in 100 ml of

ethanol was refluxed for 3 hours. The solvent was evaporated and the residue recrystallized from ethanol, giving 2.8 g of the desired product, mp 166—168°C.

Example 8

4′-(p-Chlorophenylsulfonyl)acetanilide

A mixture of 70.1 g of N-acetylsulfanilyl chloride and 60 g of aluminum chloride in 200 ml of *p*-chlorobenzede was heated until the evolution of hydrogen chloride ceased and then refluxed for 2 hours. The chlorobenzene was removed by decantation and the residue diluted with ether. The resulting gummy solid was recovered by filtration and treated with dilute hydrochloric acid and ethanol. The resulting solid was collected, dissolved in hot ethanol and concentrated, giving 19.31 g of the desired product as pale grey crystals, mp 187.5—188°C.

Example 9

2-Amino-4′-(p-chlorophenylsulfonyl)-acetanilide hydrochloride

A mixture of 5.14 g of 4′-(p-chlorophenyl-sulfonylacetanilide, 1.42 g of chloroacetyl chloride, 26 ml of dioxane and 14 ml of methoxyethyl ether was heated with stirring at 90—100°C for 2½ hours, then worked up, giving 7.9 g of 2-chloro-4′[p-(p-chlorophenylsulfonyl)phenyl]acetanilide.

A mixture of 6.0 g of the above compound and 120 ml of liquid ammonia was heated at 140°C in a sealed bomb for several hours, then opened and allowed to stand overnight. The mixture was treated with acetone, filtered, evaporated and crystallized from hexane with refrigeration. Further recrystallization gave 1.8 g of the desired product after conversion to the hydrochloride salt, mp 143—145°C.

Example 10

[p-(p-Chlorophenylsulfonyl)anilino]acetonitrile

A mixture of 43.5 g of p-chlorothiophenol, 47.1 g of *p*-chloronitrobenzene, 39.0 g of sodium carbonate, 120 ml of water and 150 ml of ethanol was heated at 100°C for 19 hours, giving 80.0 g of 4-chloro-4′-nitrodiphenylsulfide, which was then dissolved in a mixture of 400 ml of acetic acid and 100 ml of 30% hydrogen peroxide and stirred at 100°C for one hour, giving 80.2 g of the corresponding sulfonyl derivative.

The sulfonyl derivative was catalytically hydrogenated to the corresponding amino derivative.

A 16.1 g portion of the amino derivative, 4.98 g of bromoacetanonitrile, 9.0 g of sodium bicarbonated and 18 ml of 1-methyl-2-pyrrolidinone was mixed, stirred at 106°C under nitrogen for 3 hours and then quenched with ice water giving 11.8 g of the desired product as tan crystals, mp 130—132°C.

Example 11

5-[[*p*-(*p*-Chlorophenylsulfonyl)anilino]methyl]-1H-tetrazole

A reaction mixture comprising 6.14 g of [*p*-(*p*-chlorophenylsulfonyl)anilino]acetonitrile, 1.43 g of sodium azide, 30 mg of lithium chloride, 1.18 g of ammonium chloride and 10 ml of dimethylformamide was stirred at 90°C for 34 hours then filtered. The solvent was removed *in vacuo*, the residue suspended in 50 ml of water and acidified with hydrochloric acid to pH 2. The resulting solid was collected and recrystallized from acetic acid, giving 5.4 g of the desired product as tan crystals, mp 102—105°C.

Example 12

4-Fluoro-4′-(2-aminoacetamido)diphenylsulfone

4-Amino-4′-fluorodiphenylsulfone was prepared from *p*-chloronitrobenzene and *p*-chloronitrobenzene by converting the sulfide to the sulfone and reducing the nitro substituent to the amino derivative by the procedures giving in Example 10. A 15.0 g portion of the sulfone was then stirred in 200 ml of dichloromethane in an ice bath and 9.18 ml of triethylamine were added. A solution of 5.26 ml of chloroacetyl chloride were added, dichloromethane was added at such a rate as to maintain the temperature below 25°C. The ice bath was removed, the mixture stirred for one hour at room temperature then washed twice with water, treated with charcoal, dried and concentrated to a solid, giving 13.7 g of 4-fluoro-4′-(chloroacetamido)diphenylsulfone.

An 11.0 g portion of this solid was combined with 2.4 g of sodium azide in 50 ml of dimethylsulfoxide, stirred overnight, poured into 500 ml of ice and water and the solid collected. This solid was recrystallized from 150 ml of toluene, giving 9.8 g of 2-azido-N-[4-(4-fluorophenylsulfonyl)phenyl]acetamide.

A 2.0 g portion of this azide was dissolved in 40 ml of dioxane and subjected to hydrogenation with Raney nickel catalyst for 1.5 hours. The mixture was filtered, the filtrate concentrated to a residue and recrystallized from ethanol giving 944 mg of the desired product as white crystals, mp 181—184°C.

Example 13

N-[4-(4-Fluorophenylsulfonyl)-phenyl]acetamide

A 2.51 g portion of 4-amino-4′-fluorodiphenylsulfone was slurried in 100 ml of dichloromethane. A 1.84 ml portion of triethylamine was added and the mixture was cooled in an ice bath. A 1.05 ml portion of acetyl chloride in 60 ml of dichloromethane was added dropwise over 15 minutes, the ice bath was removed and the mixture stirred at room temperature for 48 hours. The solution was washed successively with water, sodium bicarbonate solution, water and sodium chloride solution then dried and concentrated to a solid.

The solid was recrystallized from 125 ml of toluene, giving 1.6 g of the desired product as white crystals, mp 181—183°C.

Example 14

N-[4-(4-Bromophenylsulfonyl)phenyl]acetamide

A mixture of 28.35 g of 4-bromothiophenol, 23.55 g of 4-chloronitrobenzene, 19 g of sodium carbonate, 100 ml of ethanol and 75 ml of water was heated at 100°C for 4 hours, then diluted with 500 ml of water. The resulting yellow solid was collected, slurried in a mixture of 250 ml of acetic acid and 100 ml of water, heated at 100°C for 2 hours cooled and diluted with 500 ml of water. The white crystals were collected, giving 46.0 g of 4-bromo-4'-nitrodiphenylsulfone.

A 37.6 g portion of the above nitro derivative was reduced to the corresponding amino derivative.

A 3.11 g portion of the above amino derivative was slurried in 75 ml of dichloromethane, 2.15 ml of triethylamine was added and the mixture was cooled in an ice bath. A 1.13 ml portion of acetylchloride in 40 ml of dichloromethane was added dropwise over 30 minutes, the ice bath was removed and the mixture was stirred over 48 hours. The solution was worked up as described in Example 13, giving 2.5 g of the desired product as tan crystals, mp 195—199°C.

Example 15

N-[4-(3-Chlorophenylsulfonyl)-phenyl]acetamide

To a cooled solution of 3.0 g of 3-chlorophenyl-4'-aminophenylsulfone in 60 ml of dichloromethane was added simultaneously and dropwise 0.92 ml of acetylchloride and 1.72 ml of triethylamine over a 10 minute period. The reaction was stirred at room temperature for 2 hours, then allowed to stand overnight and poured into 60 ml of water. The organic layer was separated and evaporated to a residue. The residue was crystallized from toluene, giving 3.2 g of the desired product, mp 149—150°C.

Example 16

N-[4-[(4-Fluorophenyl)thio]phenyl]acetamide

4-Fluoro-4'-nitrodiphenylsulfide was hydrogenated in dioxane, using Raney nickel catalyst, giving 4-[(4-fluorophenyl)thio]-benzenamine.

A 2.19 g portion of this amine was dissolved in 50 ml of dichloromethane, cooled in an ice bath and treated with 1.52 g of triethylamine. The mixture was then treated dropwise with a solution of 1.18 g of acetyl chloride in 20 ml of dichloromethane over 20 minutes. This mixture was stirred at room temperature overnight, then the solution was washed successively with water, saturated aqueous sodium bicarbonate, water and brine, dried over magnesium sulfate, filtered and concentrated to dryness. The residue was recrystallized from methanol-water, giving 2.26 g of the desired product as colorless crystals, mp 140—141°C.

Example 17

N-[4-[Fluorophenyl)sulfinyl]phenyl]acetamide

A solution of 1.3 f of n-[4-[(4-fluorophenyl]-thio]phenyl]acetamide in 75 ml of glacial acetic acid was heated to 50—70°C, treated with 0.17 g of 30% hydrogen peroxide and stirred for one hour. The mixture was diluted with 5 volumes of ice water and stirred. The solid was collected, washed with water and dried, giving 1.2 g of the desired product as a colorless solid, mp 169—170°C.

Example 18

N-[4-[(4-Methoxyphenyl]thio]phenyl]acetamide

4-Methoxy-4'-nitrodiphenylsulfide was reduced to 4-[(4-methoxyphenyl)thio] benzeneamine as described in Example 16 and then further reacted as described in Example 16, giving the desired product as a pale yellow solid, mp 100—102°C.

Example 19

N-[4-[(4-Methoxyphenyl)sulfinyl]phenyl]acetamide

An 8.2 g portion of n-[4-[(4-methoxyphenyl)-thio]phenyl]acetamide was reacted as described in Example 17, giving 6.7 g of the desired product as a pale yellow solid, mp 63—65°C.

Example 20

N-[4-[(4-Chlorophenyl)thio]phenyl]acetamide

4-Chloro-4'-nitrodiphenylsulfide was reduced to 4-[(4-chlorophenyl)thio]benzeneamine and then further reacted as described in Example 16, giving the desired product as beige crystals, mp 140—141°C.

Example 21

N-[4-[(4-Chlorophenyl)sulfinyl]phenyl]acetamide

An 8 g portion of N-[4-[(4-chlorophenyl)thio]phenyl]acetamide was reacted as described in Example 17, giving 7 g of the desired product as a colorless solid, mp 160—162°C.

Example 22

N-[4-[(4-Bromophenyl)thio]phenyl]acetamide

4-Bromo-4′-nitrodiphenylsulfide was reduced to 4-[(4-bromophenyl)thio]benzeneamine and further reacted as described in Example 16, giving the desired product as beige crystals, mp 153—155°C.

Example 23

N-[4-[(4-Bromophenyl)sulfinyl]phenyl]acetamide

A 10 g portion of N-[4-[(4-bromophenyl]thio]phenyl]acetamide was reacted as described in Example 17, giving 2.31 g of the desired product as beige crystals, mp 190—191°C.

Example 24

N-[4-[(4-Fluorophenyl)sulfonyl]phenyl]-2-methylpropanamide

A 2.51 g portion of 4-[4-fluorophenyl)sulfonyl]benzeneamine was reacted as described in Example 16, using propionyl chloride in place of acetyl chloride giving 2.74 g of the desired product, mp 112—115°C.

Example 25

N-[4-(Phenylthio)-phenyl)acetamide

4-Nitrodiphenyl sulfide was reduced to 4-(phenylthio)benzeneamine and further reacted as described in Example 16, giving the desired product as orange crystals, mp 142—144°C

Example 26

N-[4-(Phenylsulfinyl)phenyl]acetamide

A 2.43 g portion of N-[4-(phenylthio)phenyl]acetamide was reacted as described in Example 17, giving 1.1 g of the desired compound as a colorless solid, mp 135—137°C.

Example 27

p-(p-Fluorophenylsulfonyl)aniline

A mixture comprised of 38.4 g of 4-fluorothio-phenol, 47.1 g of p-chloronitrobenzene, 39 g of sodium carbonate, 120 ml of water and 150 ml of ethanol was stirred at 100°C for 19 hours and then poured into 500 ml of water. The solid was collected, washed with water and recrystallized from ethanol, giving 66.4 g of 4-fluoro-4′-nitrodiphenyl sulfide.

A 30 g portion of the above compound was added to a mixture of 200 ml of glacial acetic acid and 50 ml of 30% hydrogen peroxide and stirred at 100°C for one hour. The mixture was cooled, 15 ml of water was added and the mixture was refrigerated overnight. The crystals were collected giving 32.7 g of 4-fluoro-4′-nitrodiphenyl sulfone.

A mixture of 10.0 g of the above sulfone, 200 ml of dioxane and 3 ml of Raney nickel catalyst in water was hydrogenated in a Parr apparatus until hydrogen uptake was complete. The catalyst was removed by filtration. The filtrate was concentrated *in vacuo* and the residue crystallized from 200 ml of ethanol, giving 7.8 g of the desired product as colorless crystals, mp 206—208°C.

Example 28

p-(*o*-Nitrophenylthio)aniline

To a stirred mixture of 12.51 g of p-aminothio-phenol and 11.6 g of sodium carbonate in 200 ml of ethanol and 50 ml of water was added 15.7 g of 1-chloro-2-nitrobenzene. The mixture was refluxed with stirring for 4 hours, then cooled and filtered. The filtrate was treated with dichloromethane and water and the resulting solid was crystallized from toluene giving 18.57 g of the desired product, mp 78—82°C.

Example 29

p-(2,4,5-Trichlorophenylsulfonyl)aniline

A mixture of 32.03 g of 2,4,5-tricholorthiophenol, 19.5 g of sodium carbonate and 23.55 g of 4-chloronitrobenzene in water and ethanol was reacted as described in Example 27, giving 30 g of 2,4,5-trichloro-4′-nitrodiphenyl sulfide.

A 20 g portion of the above sulfide was reacted with 30% hydrogen peroxide and glacial acetic acid as described in Example 27, giving 19.0 g of 2,4,5-trichloro-4′-nitrodiphenyl sulfone.

A 15.0 g portion of the above sulfone was reduced in dioxane as described in Example 27, giving 11.3 g of the desired product, mp 195—197°C.

Example 30

p-(p-Bromophenylsulfonyl)aniline

A mixture of 56.7 g of 4-bromothiophenol, 47.1 g 4-chloronitrobenzene and 39 g of sodium carbonate in water and ethanol was reacted as described in Example 27, giving 78.3 g of 4-bromo-4′-nitrodiphenyl sulfone.

A 30 g portion of the above sulfone was reduced in dioxane as described in Example 27, giving 20.0 g of the desired product as white crystals, mp 198—200°C.

Example 31

p-(2,4,5-Trichlorophenylthio)aniline

A 5 g portion of 2,4,5-trichloro-4′-nitrodiphenyl sulfide in dioxane was reduced as decribed in Example 27, giving 2.0 g of the desired product as pink crystals, mp 118—120°C.

Example 32

p-(2,5-Dichlorophenylthio)aniline

A mixture of 25 g of 2,5-dichlorobenzenethiol, 19.5 g of sodium carbonate and 22.05 g of 1-chloro-4-nitrobenzene in ethanol and water was reacted as described in Example 27, giving 32.5 g of 2.5-dichloro-4′-nitrodiphenyl sulfide.

A 10.0 g portion of the above compound was reduced in dioxane as described in Example 27, giving 8.5 g of the desired product as an off-white solid, mp 67—69°C.

Example 33

p-(m-Chlorophenylsulfonyl)aniline

3-Chloro-4′-nitrodiphenylsulfone was prepared from 3-chlorothiophenol, *p*-chloronitrobenzene and sodium carbonate in ethanol-water, followed by reaction with hydrogen peroxide in glacial acetic acid, all as described in Example 27.

Example 34

p-(3,4-Dichlorophenylthio)aniline

A mixture of 25 g of 3,4-dichlorobenzenethiol, 19.5 g of sodium carbonate, 22.05 g of 1-chloro-4-nitrobenzene, 100 ml of ethanol and 75 ml of water was reacted as described in Example 27, giving 34.8 g of 3,4-dichloro-4′-nitrodiphenylsulfide.

A 15.0 g portion of the above compound in dioxane was reduced as described in Example 27, giving 13.3 g of the desired product as a yellow solid, mp 52—55°C.

Example 35

p-(p-Methoxyphenylsulfonyl)aniline

A 20 g portion of 4-methoxy-4′-nitrodiphenyl-sulfide was reacted with hydrogen peroxide and glacial acetic acid as described in Example 27, giving 21.3 g of 4-methoxy-4′-nitrodiphenylsulfone.

A 17.0 g portion of the above compound in dioxane was reduced as described in Example 27, giving 14.0 g of the desired product as a white solid, mp 149—151°C.

Example 36

p-(3,4-Dichlorophenylsulfonyl)aniline

A 15 g portion of 3,4-dichloro-4′-nitrodiphenyl-sulfide was reacted with hydrogen peroxide in glacial acetic acid as described in Example 27, giving 15.9 g of 3,4-dichloro-4′-nitrodiphenylsulfone.

A 12.0 g portion of the above compound in dioxane was reduced as described in Example 27, giving 1.9 g of crude product, which was recrystallized from ethanol, giving 5.5 g of the desired product as white crystals, mp 214—216°C.

Example 37

3-Chloro-4-(p-chlorophenylsulfonyl)aniline

A mixture of 25.9 g of 4-chlorothiophenyl, 34.4 g of 1,2-dichloro-4-nitrobenzene, 19.5 g of sodium carbonate, 100 ml of ethanol and 75 ml of water was reacted as described in Example 27, giving 30.1 g of 2′,3-dichloro-4′-nitrodiphenyl sulfide.

A 15.0 g portion of the above compound was reacted with hydrogen peroxide and glacial acetic acid as described in Example 27, giving 16.5 g of 2′,3-dichloro-4′-nitrodiphenyl sulfone.

A 13.0 g portion of the above sulfone in dioxane was reduced as described in Example 27, giving 8.7 g of the desired product as pink crystals, mp 175—177°C.

Example 38

p-(p-Chlorophenylsulfonyl)aniline

This compound is commercially available from Alfred Bader Chemical Company.

Example 39

2-[!p-(p-Aminophenylsulfonyl)phenyl]amino]ethanol

This compound is commercially available from Alfred Bader Chemical Company.

Example 40

4-Acetylamino-4′-aminodiphenylsulfone

This compound is commercially available from Alfred Bader Chemical Company.

Example 41

p-[(p-Bromophenyl)thio]benzeneamine

A 25.0 g portion of 4-bromo-4'-nitrodiphenylsulfide was dissolved in 150 ml of dioxane. A solution of Raney nickel catalyst in water was added and the mixture was hydrogenated for 2 hours, then filtered through diatomaceus earth. The filtrate was concentrated in vacuo to an oil which was dissolved in 100 ml of ether and concentrated in vacuo, giving 21.2 g of the desired product as pale yellow, mp 64—66°C.

Example 42

p-[(p-Methoxyphenyl)thio]benzeneamine

A mixture of 28 g of 1-chloro-4-nitrobenzene, 25 g of 4-methoxybenzenthiol, 19.5 g of sodium carbonate, 100 ml of ethanol and 75 ml of water was heated at 100°C. for 4 hours, then poured into 600 ml of cold water. The solid was collected and crystallized from 150 ml of ethanol, giving 39.8 g of 4-methoxy-4'-nitrodiphenylsulfide.

A 15 g portion of the above compound in dioxane was reduced as described in Example 43, giving 12.7 g of the desired product as a pale-yellow solid, mp 89—91°C.

Example 43

*p*-[(*p*-Fluorophenyl)thio]benzeneamine

A 7 g portion of 4-fluoro-4'-nitrodiphenylsulfide in 120 ml of *p*-dioxane was reduced as described in Example 17, giving 5.5 g of the desired product as a pale pink solid, mp 55—57°C.

Example 44

p-[(p-Chlorophenyl)thio]benzeneamine

A 13.3 g portion of 4-chloro-4'-nitrodiphenylsulfide in 150 ml of dioxane was reduced as described in Example 43, giving 9.62 g of the desired product as light pink crystals, mp 61—62°C.

Example 45

*p*-[(*p*-Fluorophenyl)thio]-N,N-dimethylbenzeneamine

A suspension of 12.5 g of 4-fluoro-4'-nitrodiphenylsulfide, 4.5 g of 37% formaldehyde, 1 g of sodium acetate and 6 g of Raney nickel catalyst in 130 ml of ethanol was hydrogenated in a Parr shaker for 16 hours. The mixture was filtered and the filtrate treated with twice its volume of water giving a precipitate which was collected and recrystallized from 40 ml of methanol, giving the desired product, mp 70—72°C.

Example 46

*p*-[(*p*-Fluorophenyl)sulfinyl]-N.N-dimethylbenzeneamine

A 2.47 g portion of *p*-[(p-fluorophenyl)thio]-N,N-dimethylbenzeneamine was dissolved in 40 ml of glacial acetic acid and clarified by filtration. The filtrate was treated at 40°C with 1.4 ml of 30% hydrogen peroxide, with stirring for one hour. Three volumes of water were added and the mixture was stirred overnight. The white solid was collected, giving 1.62 g of the desired product, mp 113—115°C.

Example 47

*p*-[(*p*-Fluorophenyl)sulfinyl]benzeneamide

A 2.19 g portion of *p*-[(*p*-fluorophenyl)thio]benzeneamine was dissolved in 50 ml of dichloromethane. This solution was treated with 1.52 g of triethylamine while cooled in an ice bath and then treated dropwise with a solution of 1.18 g of acetyl chloride in 20 ml of dichloromethane over a period of 20 minutes. The mixture was stirred at room temperature overnight, then washed successively with water, saturated aqueous sodium bicarbonate, water and sodium chloride solution, then dried over magnesium sulfate, filtered and concentrated to dryness, giving 2.68 g of *p*-[(*p*-fluorophenyl)thio)benzeneacetamide.

A 1.3 g portion of the above compound in 25 ml of acetic acid at 50—70°C was treated with 0.17 g of 30% hydrogen peroxide and stirred for one hour. The mixture was diluted with 5 volumes of ice water, giving 1.2 g of *p*-[(*p*-fluorophenyl)sulfinyl]benzeneacetamide.

A solution of 1.38 g of the above sulfonyl derivative in 50 ml of ethanol containing 5 ml of 7N hydrochloric acid in isopropanol was heated at reflux for ½ hour and then evaporated to dryness. The residue was taken in water, giving an oil which was crystallized from methanol and water, giving 0.2 g of the desired product as a colorless solid, mp 145—147°C.

Example 48

*p*-(Phenylthio)benzeneamine

A 15 g portion of 4-nitrodiphenylsulfide was hydrogenated with Raney nickel catalyst in dioxane, giving 7.8 g of the desired product as yellow crystals, mp 94—96°C.

Example 49

*p*-[(*p*-Chlorophenyl)sulfinyl]benzeneamine

*p*-[(*p*-Chlorophenyl)thio]benzenamine was converted to *p*[(*p*-chlorophenyl)thio)benzeneacetamide by

the procedure described in Example 49 and then to *p*-[(*p*-chlorophenyl)sulfinyl]benzeneacetamide and then (still by the procedure of Example 49) converted to the desired product, colorless crystals, mp 161—162°C.

Example 50

*p*-[(*p*-Methoxyphenyl)sulfinyl]benzeneamine

Following the procedure of Example 49, *p*-[(*p*-methoxyphenyl)thio]benzeneamine was converted to *p*-[(*p*-methoxyphenyl)thio]benzeneacetamide, which was converted to *p*-[(p-methoxyphenyl)-sulfinyl]benzeneacetamide and finally to the desired product, a colorless solid, mp 135—140°C.

**Claims**

1. Use of a compound of the following formula:

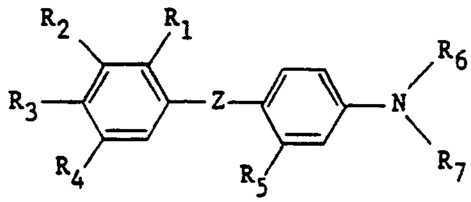

wherein $R_1$ is hydrogen, nitro or chloro; $R_2$ is hydrogen or chloro; $R_3$ is hydrogen, fluoro, chloro, bromo, nitro, alkoxy ($C_1$—$C_3$), acetylamino, B-hydroxyethylamino or —$NHCOCH_2NHCH_3$; $R_4$ is hydrogen or chloro; $R_5$ is hydrogen or chloro; $R_6$ is hydrogen or alkyl ($C_1$—$C_3$); $C_7$ is hydrogen, alkyl ($C_1$—$C_3$), —$COCH_2NH_2$, —$COCH_2NHCH_3$, —$COCH_2CH_2Cl$, —$COCH_2Cl$, —$CH_2CON$, (5-1H-tetrazolyl) methyl or

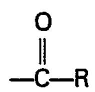

wherein R is alkyl having up to 4 carbon atoms; and Z is thio (—S—), sulfinyl (—SO—) or sulfonyl ($SO_2$—); with the proviso that the phenyl ring bearing the $R_1$, $R_2$, $R_3$ and $R_4$ substituents is mono-substituted except for polychloro substitution; and the pharmacologically acceptable acid-addition salts thereof; in association with a pharmaceutically acceptable carrier for the manufacture of a medicament for modulating the immune response system in warm-blooded animals.

2. The use according to claim 1, wherein the compound is 3-chloro-4'-(*p*-nitrophenylthio)propionanilide.

3. The use according to claim 1, wherein the compound is 4'-(*p*-chlorophenylsulfonyl)acetanilide.

4. The use according to claim 1, wherein the compound is N-[4-(4-fluorophenylsulfonyl)phenyl]-acetamide.

5. The use according to claim 1, wherein the compound is *p*-(2,5-dichlorophenylthio)aniline.

6. A compound of the following formula:

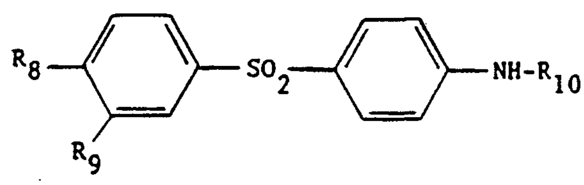

wherein $R_8$ is hydrogen, fluoro, chloro or bromo and $R_9$ is hydrogen or chloro with the proviso that one of $R_8$ and $R_9$ must be hydrogen but $R_8$ and $R_9$ may not both be hydrogen and $R_{10}$ is —$CH_2CN$, —$COCH_2NH_2$ or (5-1H-tetrazolyl)methyl; and the pharmacologically acceptable acid-addition salts thereof.

7. The compound according to Claim 5 wherein $R_8$ is chloro, $R_9$ is hydrogen, and $R_{10}$ is —$CH_2CN$; [*p*-(*p*-chlorophenylsulfonyl)anilino]acetonitrile.

8. The compound according to Claim 5 wherein $R_8$ is hydrogen, $R_9$ is chloro, and $R_{10}$ is —$COCH_2NH_2$; 2-amino-4'-(*m*-chlorophenylsulfonyl)acetanilide.

9. The compound according to Claim 5 wherein $R_8$ is fluoro, $R_9$ is hydrogen, and $R_{10}$ is —$COCH_2NH_2$; 4-fluoro-4'-(2-aminoacetamido)diphenylsulfone.

10. The compound according to Claim 5 wherein $R_8$ is chloro, $R_9$ is hydrogen, and $R_{10}$ is (5-1H-tetrazolyl)-methyl; 5-[*p*-(*p*-chlorophenylsulfonyl)anilinomethyl]-1H-tetrazole.

**Patentansprüche**

1. Verwendung einer Verbindung der folgenden Formel

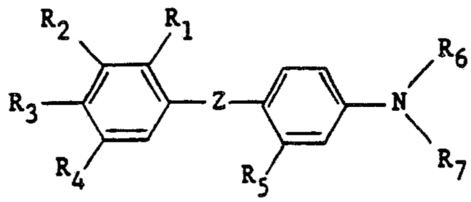

wobei $R_1$ für Wasserstoff, Nitro oder Chlor steht; $R_2$ für Wasserstoff oder Chlor steht; $R_3$ für Wasserstoff, Fluor, Chlor, Brom, Nitro, Alkoxy ($C_1$—$C_3$), Acetylamino, β-Hydroxyäthylamino oder —$NHCOCH_2NHCH_3$ steht; $R_4$ für Wasserstoff oder Chlor steht; $R_5$ für Wasserstoff oder Chlor steht; $R_6$ für Wasserstoff oder Alkyl ($C_1$—$C_3$) steht; $R_7$ für Wasserstoff, Alkyl ($C_1$—$C_3$) —$COCH_2NH_2$, —$COCH_2NHCH_3$, —$COCH_2CH_2Cl$, —$COCH_2Cl$, —$CH_2CN$, (5-1H-Tetrazolyl)methyl oder

O
‖
—C—R steht,

wobei R für Alkyl mit bis zu 4 Kohlenstoffatomen steht; und z steht für Thio (—S—), Sulfinyl- (—SO—) oder Sulfonyl ($SO_2$—); mit der Maßgabe, daß der die Substituenten $R_1$, $R_2$, $R_3$ und $R_4$ tragende Phenylring monosubstituiert ist, ausgenommen eine Polychlorsubstitution; und die pharmakologisch akzeptablen Säureadditionssalze derselben; gemeinsam mit einem pharmazeutisch akzeptablen, Träger zur Herstellung eines Medikaments zur Modulierung des Immunresponse-Systems bei Warmblütern.

2. Verwendung nach Anspruch 1, wobei die Verbindung 3-Chlor-4'-(*p*-nitrophenylthio)propionanilid ist.

3. Verwendung nach Anspruch 1, wobei die Verbindung 4'-(*p*-Chlorophenylsulfonyl)acetanilid ist.

4. Verwendung nach Anspruch 1, wobei die Verbindung N-[4-(4-Fluorophenylsulfonylphenyl]acetamid ist.

5. Verwendung nach Anspruch 1, wobei die Verbindung *p*-(2,5-Dichlorphenylthio)anilin ist.

6. Eine Verbindung der folgenden Formel

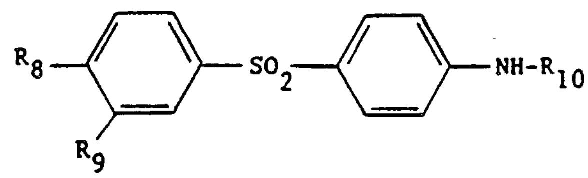

wobei $R_8$ für Wasserstoff, Fluor, Chlor oder Brom steht und $R_9$ für Wasserstoff oder Chlor steht, mit der Maßgabe, daß einer der Reste $R_8$ und $R_9$ Wasserstoff sein muß, jedoch $R_8$ und $R_9$ nicht beide für Wasserstoff stehen können und $R_{10}$ für —$CH_2CN$, —$COCH_2NH_2$ oder (5-1H-Tetrazolyl)methyl steht; und die Pharmakologisch akzeptablen Säureadditionssalze derselben.

7. Verbinding nach Anspruch 5, wobei $R_8$ für Chlor steht, $R_9$ für Wasserstoff steht und $R_{10}$ für —$CH_2CN$ steht; [*p*-(p-Chlorphenylsulfonyl)anilino]acetonitril.

8. Verbindung nach Anspruch 5, wobei $R_8$ für Wasserstoff steht, $R_9$ für Chlor steht und $R_{10}$ für —$COCH_2NH_2$ steht, 2-Amino-4'-(m-chlorphenylsulfonyl)acetanilid.

9. Verbindung nach Anspruch 5, wobei $R_8$ für Fluor steht, $R_9$ für Wasserstoff steht und $R_{10}$ für —$COCH_2NH_2$ steht; 4-Fluor-4'-(2-aminoacetamido)diphenylsulfon.

10. Verbindung nach Anspruch 5, wobei $R_8$ für Chlor steht, $R_9$ für Wasserstoff steht und $R_{10}$ für (5-1H-Tetrazolyl)methyl steht; 5-[*p*-(*p*-Chlorphenylsulfonyl)anilinomethyl]-1H-tetrazol.

**Revendications**

1. Emploi d'une composé répondant à la formule suivante:

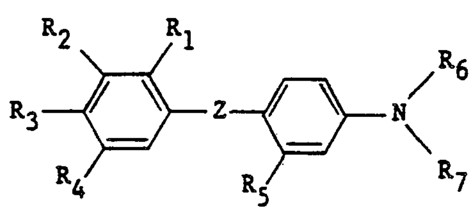

dans laquelle $R_1$ est un hydrogène, un nitro ou un chloro; $R_2$ est un hydrogène ou un chloro; $R_3$ est un hydrogène, une fluoro, un chloro, un bromo, un nitro, un alcoxy ($C_1$—$C_3$), un acétylamino, un β-hydroxyéthylamino ou $—NHCOCH_2NHCH_3$; $R_4$ est un hydrogène ou un chloro; $R_5$ est un hydrogène ou un chloro; $R_6$ est un hydrogène ou un alkyle ($C_1$—$C_3$); $R_7$ est un hydrogène, un alkyle ($C_1$—$C_3$), $—COCH_2NH_2—$, $—COCH_2NHCH_3$, $—COCH_2CH_2Cl$, $—COCH_2Cl$, $—CH_2CN$, (5-1H-tétrazolyl)méthyle ou

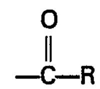

où R est un alkyle ayant jusqu'à 4 atomes de carbone, et Z est un thio (—S—), un sulfinyle (—SO—)ou un sulfonyle ($—SO_2—$); sous réserve que le cycle phényle portant les substitution $R_1$, $R_2$, $R_3$ et $R_4$ est monosubstitué à l'exception de la substitution polychloro; et leurs sels d'additions d'acides convenant en pharmacologie; en association avec un support convenant en pharmacie, pour la fabrication d'un médicament pour moduler le système de réponse immunitaire des animaux à sang chaud.

2. L'emploi selon la revendication 1 où le composé est le 3-chloro-4'-(p-nitrophénylthio)propionanilide.

3. L'emploi selon la revendication 1 où le composé est le 4'-(p-chlorophénylsulfonyl)acétanilide.

4. L'emploi selon la revendication 1 où le composé est le N-[4-(4-fluorophénylsulfonyl)-phényl]acétamide.

5. L'emploi selon la revendication 1 où le composé est le p-(2,5-dichlorophénylthio)aniline.

6. Un composé répondant à la formule suivante:

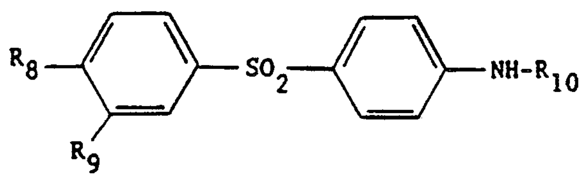

dans laquelle $R_8$ est un hydrogène, un fluoro, un chloro ou un bromo et $R_9$ est un hydrogène ou un chloro, sous réserve qu'un de $R_8$ et $R_9$ doit être un hydrogène, mais $R_8$ et $R_9$ ne peuvent pas tous deux être un hydrogène et $R_{10}$ est $—CH_2CN$, $—COCH_2NH_2$ ou (5-1H-tétrazolyl)méthyle; et leurs sels d'addition d'acides convenant en pharmacologie.

7. Le composé selon la revendication 5 où $R_8$ est un chloro, $R_9$ est un hydrogène et $R_{10}$ est $—CH_2CH$; le [p-(p-chlorophénylsulfonyl)anilino]acétonitrile.

8. Le composé selon la revendication 5 où $R_8$ est un hydrogène, $R_9$ est un chloro et $R_{10}$ est $—COCH_2NH_2$; le 2-amino-4'-(m-chlorophénylsulfonyl)acétanilide.

9. Le composé selon la revendication 5 où $R_8$ est un fluoro, $R_9$ est un hydrogène et $R_{10}$ est $—COCH_2NH_2$; la 4-fluoro-4'-(2-amino-acétamido)diphénylsulfone.

10. Le composé selon la revendication 5 où $R_8$ est un chloro, $R_9$ est un hydrogène et $R_{10}$ est un (5-1H-tétrazolyl)méthyle; le 5-[p-(p-chlorophénylsulfonyl)anilinométhyl]-1H-tétrazole.